# EUROPEAN PATENT APPLICATION

(11) **EP 0 791 393 A1**
(43) Date of publication of application: **27.08.1997**
(21) Application number: 97101012.9
(22) Date of filing: 23.01.1997
(51) Int. Cl.: B01J 23/96, C07C 57/66, C07C 51/58

(54) **Palladium catalyst recovery**

(30) Priority: 23.02.1996 US 606139
(71) Applicant: E.I. DU PONT DE NEMOURS AND COMPANY, Wilmington Delaware 19898 (US)
(72) Inventor: Chiang, Chen-Chou, Hockessin, Delaware 19707-1901 (US)
(74) Representative: Abitz, Walter, Dr.-Ing.

(57) **Abstract**

Palladium is recovered from a mixture of high boiling organic compounds by extracting with an aqueous solution of hydrochloric acid which may contain an oxidizing agent, then distilling off the hydrochloric acid and decomposing the oxidizing agent, converting the palladium into crotyl palladium chloride, and extracting the palladium with chlorobutene from the aqueous phase.

## Description

### Field of the Invention:

This invention relates to the recovery of palladium catalyst from a mixture of organic compounds having a high boiling point. Said mixture of organic compounds may be the still heels obtained by the distillation of 3-pentenoylchloride from a reaction mixture containing palladium catalyst residue.

### Background of the Invention:

Bunel et al. U.S. Patent 5,288,903 discloses the preparation of pentenoylchloride by the carbonylation of chlorobutenes using a palladium catalyst.

Bunel et al. U.S. Patent 5,399,753 discloses a process for the recovery of palladium catalyst from the still heels obtained by the distillation of 3-pentenoylchloride from a reaction mixture containing palladium catalyst residue.

The present invention is an alternative to the process of the '753 Patent. The process of the present invention is more efficient, recovers more of the palladium contained in the mixture of organic compounds, operates under milder conditions, and consumes less energy.

### Summary of the Invention:

The present invention is a process for the recovery of palladium catalyst from a mixture also containing high boiling organic compounds which comprises (a) extracting palladium from said mixture by contacting the mixture at a temperature in the range of 20 to 110°C with an aqueous solution of hydrochloric acid where the hydrochloric acid concentration is in the range of 5% to 37% by weight or with such an aqueous solution of hydrochloric acid and an oxidizing agent, (b) allowing the components of step a to form two phases namely an organic phase and an aqueous phase, said aqueous phase containing dissolved palladium, (c) separating the two phases, (d) heating the aqueous phase to distill off most of the hydrochloric acid and to decompose any oxidizing agent, (e) mixing the product of step d with chlorobutene and heating the thus formed mixture under a carbon monoxide atmosphere or a carbon monoxide/hydrogen atmosphere to convert the palladium to crotyl palladium chloride, (f) allowing the product of step e to form two phases, namely an aqueous phase and an organic phase, said organic phase containing chlorobutene and crotyl palladium chloride, (g) and separating the organic phase of step f from the aqueous phase of step f. If desired, the organic phase obtained in step g may be dried, for example, by the use of a molecular sieve, or the addition of absorbents, adsorbents, or desiccants.

In one aspect of the invention the high boiling organic compounds are still heels remaining after the distillation of 3-pentenoylchloride from an organic mixture.

A suitable oxidizing agent for use in the process is selected from the group consisting of hydrogen peroxide, nitric acid, potassium permanganate, ozone, air, and a halogen gas.

Preferably, the aqueous solution of hydrochloric acid used in step a contains 12% to 37% by weight hydrochloric acid.

A preferred oxidizing agent is hydrogen peroxide, and it is preferably present in the aqueous solution of hydrochloric acid in the amount of 0.3 to 2% by weight.

Sometimes prior to step a, the mixture containing high boiling organic compounds is treated with concentrated hydrochloric acid to extract some palladium.

### Detailed Description:

3-pentenoylchloride may be prepared by the reaction of chlorobutene and carbon monoxide in the presence of a palladium catalyst. See Bunel et al. U.S. Patent 5,288,903, which is incorporated herein by reference. After separation of 3-pentenoylchloride the residue contains high boiling organic compounds and palladium catalyst residue. The high boiling compounds may be the result of side reactions in the preparation of chlorobutene by the hydrochlorination of butadiene, in which case the most abundant by-product is C₈Cl compounds and C₈Cl₂ compounds, and/or from the side reactions in the carbonyalation of chlorobutene, in which case the most abundant product is 3-pentenoic acid, 3-pentenoic acid anhydride, chlorinated 3-pentenoic acid anhydride, and chlorovaleric acid. Such compounds have boiling points above 130°C at atmospheric pressure are "high boiling compounds" as that term is used herein. The palladium catalyst residue is recovered, according to the present invention by extracting the palladium with an aqueous solution of hydrochloric acid or a mixture of such an aqueous solution of hydrochloric acid and an oxidizing agent. Multiple extractions may be advantageously employed: extractions using an aqueous solution of hydrochloric acid may be followed by extractions using an aqueous solution of hydrochloric acid and an oxidizing agent. The concentration of the hydrochloric acid should be in the range of 5% to 37% by weight. Suitable oxidizing agents include, hydrogen peroxide, nitric acid, potassium permanganate, ozone, air, and a halogen gas. The extraction may be carried out at a temperature of 20 to 110°C.

It is often desirable to add a solvent to the mixture of high boiling compounds and palladium catalyst before the extraction with aqueous hydrochloric acid. The solvent makes the mixture easier to handle and speeds the extraction. Suitable solvents include: toluene, cyclohexene, chlorobutene, and chlorotrifluoroethane.

The palladium will be extracted into the aqueous phase of hydrochloric acid. The phases are separated. (If there are multiple extractions, the several aqueous phases will usually be combined.) The aqueous phase is heated to distill off most of the hydrochloric acid and destroy the oxidizing agent. This distillation may be carried out at a temperature in the range of 30 to 150°C depending on the pressure employed. After distillation the remainder of the aqueous phase is heated with chlorobutene solvent under a carbon monoxide containing atmosphere to cause crotyl palladium chloride to form and be extracted into the organic phase. A temperature in the range of 25 to 100°C is satisfactory. The heating may be done at pressures in the range of one atmosphere to 20 atmospheres. Higher temperatures require a shorter time, but a time in the range of 5 minutes to 120 minutes is usually satisfactory. The amount of chlorobutene is not critical, but should be sufficient to prevent crotyl palladium chloride precipitation. A volume ratio of chlorobutene to aqueous phase of 0.5 to 50 is usually sufficient.

The mixture is then allowed to form two phases, and the organic phase containing chlorobutene and crotyl palladium chloride is separated from the aqueous phase.

The separated organic phase may be combined with carbon monoxide and reacted to form 3-pentenoylchloride per the Bunel et al. '903 Patent, or the crotyl palladium chloride may be recovered by evaporating the organic phase and filtration.

### Examples

### Example 1: HCl/H₂O₂ Extraction

20g of high boilers mixture (mixture of high boilers from hydrochlorination reaction and carbonylation reaction, [Pd]=16689 ppm) were combined with 20g of toluene (solvent) and 130.8g of HCl solution (18%) and 5.46g of H₂O₂ solution (30%). The mixture was mixed at 30-35°C for 20 minutes. The organic phase was separated from the HCl/H₂O₂ phase (137.9g). The Pd concentration in HCl/H₂O₂ phase was 2714 ppm.

The organic phase was combined with 130.8g of HCl solution (18%) and 5.91g of H₂O₂ solution (30%). The mixture was mixed at room temperature for 20 minutes. The organic phase was separated from the HCl/H₂O₂ phase (137.4g). The Pd concentration in HCl/H₂O₂ phase was 64 ppm.

### Example 2: Counter Current HCl/H₂O₂ Extraction

20g of high boilers mixture (mixture of high boilers from hydrochlorination reaction and carbonylation reaction, [Pd]=16296 ppm) were combined with 20g of toluene (solvent) and the HCl/H₂O₂ solution from the second step in Example 1. The mixture was mixed at 30-35°C for 20 minutes. The organic phase was separated from the HCl/H₂O₂ phase (135.5g). The Pd concentration in HCl/H₂O₂ phase was 2425 ppm.

The organic phase was combined with 130.8g of HCl solution (18%) and 5.65g of H₂O₂ solution (30%). The mixture was mixed at room temperature for 20 minutes. The organic phase was separated from the HCl/H₂O₂ phase (137.0g). The Pd concentration in HCl/H₂O₂ phase was 77 ppm.

### Example 3: HCl Evaporation/H₂O₂ Decomposition

The HCl/H₂O₂ phase from the first step of both Examples 1 and 2 were combined. The mixture was heated to 110°C to evaporate the HCl solution and to decompose the H₂O₂. The distillate was collected at room temperature. After collecting 203.2g of distillate, the evaporation was stopped. 36.1g of HCl solution was collected from the pot and its Pd concentration was 18044 ppm.

### Example 4: Back Extraction

31.3g of the HCl solution ([Pd]=18044 ppm) obtained in Example 3 was combined with 34g of chlorobutene in a round bottom flask. A sparge tube was introduced into the liquid to maintain a carbon monoxide atmosphere during the back extraction operation. The mixture was agitated and heated to 50°C for 1 hour. Then the chlorobutene phase was separated from the HCl solution. The Pd concentration in chlorobutene (31.3g) was 9072 ppm, and the Pd concentration in HCl solution was 8838 ppm.

## Claims

1. A process for the recovery of palladium catalyst from a mixture also containing high boiling organic compounds which comprises (a) extracting palladium from said mixture by contacting the mixture at a temperature in the range of 20 to 110°C with an aqueous solution of hydrochloric acid where the hydrochloric acid concentration is in the range of 5% to 37% by weight or with such an aqueous solution of hydrochloric acid and an oxidizing agent.

2. A process for the recovery of palladium catalyst from a mixture also containing high boiling organic compounds which comprises (a) extracting palladium from said mixture by contacting the mixture at a temperature in the range of 20 to 110°C with an aqueous solution of hydrochloric acid where the hydrochloric acid concentration is in the range of 5% to 37% by weight or with such an aqueous solution of hydrochloric acid and an oxidizing agent, (b) allowing the components of step a to form two phases namely an organic phase and an aqueous phase, (c) separating the two phases, (d) heating the aqueous phase to distill off most of the hydrochloric acid and to decompose any oxidizing agent, (e) mixing the product of step d with chlorobutene and heating the thus formed mixture under a carbon monoxide atmosphere or a carbon monoxide/hydrogen atmosphere to convert the palladium to crotyl palladium chloride, (f) allowing the product of step e to form two phases, namely an aqueous phase and an organic phase, said organic phase containing chlorobutene and crotyl palladium chloride, (g) and separating the organic phase of step f from the aqueous phase of step f.

3. The process of claim 2 in which the high boiling organic compounds are still heels remaining after the distillation of 3-pentenoylchloride an organic mixture, and in which the oxidizing agent is selected from the group consisting of hydrogen peroxide, nitric acid, potassium permanganate, ozone, air, and a halogen gas.

4. The process of claim 2 in which the aqueous solution of hydrochloric acid contains 12% to 37% by weight hydrochloric acid.

5. The process of claim 4 in which the oxidizing agent is present in the aqueous solution of hydrochloric acid in the amount of 0.3 to 2% by weight.

6. The process of claim 2 in which prior to step a, the mixture containing high boiling organic compounds is treated with concentrated hydrochloric acid to extract some palladium.

7. The process of claim 3 in which the oxidizing agent is hydrogen peroxide.
